# EUROPEAN PATENT APPLICATION

(11) **EP 1 847 617 A1**
(43) Date of publication of application: **24.10.2007**
(21) Application number: 07007703.7
(22) Date of filing: 16.04.2007
(51) Int. Cl.: C12Q 1/68

(54) **Detection method of SNPs**

(30) Priority: 17.04.2006 JP 2006113483; 28.04.2006 JP 2006126791
(71) Applicant: FUJIFILM Corporation, Minato-ku Tokyo (JP)
(72) Inventor: Mori, Toshihiro c/o FUJIFILM Corporation, Ashigarakami-gun Kanagawa (JP); Iwaki, Yoshihide c/o FUJIFILM Corporation, Ashigarakami-gun Kanagawa (JP)
(74) Representative: HOFFMANN EITLE

(57) **Abstract**

A method for detecting a mismatch between a target nucleic acid as a measuring obj ect and a control nucleic acid, the method comprising: (a) effecting formation of a double-stranded nucleic acid through hybridization of the control nucleic acid and the target nucleic acid; (b) allowing a mismatch binding protein to contact with the double-stranded nucleic acid and thereby to bind to a mismatched site; (c) allowing an intercalating agent which specifically recognizes the double-stranded nucleic acid and is intercalated therein, to contact with the double-stranded nucleic acid; (d) detecting the intercalating agent intercalated into the double-stranded nucleic acid; and (e) judging the presence or absence of a mismatch between the control nucleic acid and the target nucleic acid, by comparing amounts of the intercalating agent intercalated into the double-stranded nucleic acid in the absence and presence of the mismatch binding protein.

## Description

### Background of the Invention

### 1. Field of the Invention

This invention relates to a method for detecting a mismatch between a single-stranded nucleic acid as the measuring object (target nucleic acid) and a single-stranded nucleic acid of known sequence (control nucleic acid), which comprises using a mismatch binding protein. According to the method of the invention, a single base gene polymorphism (SNPs: single nucleotide polymorphism) in a DNA nucleotide sequence can be detected.

### 2. Description of the Related Art

Gene expression profile analyses and analyses of single base substitution (SNPs: single nucleotide polymorphism) are drawing attention next to the genome sequence analyses. Functions of genes and relationships between genes and diseases or drug sensitivities have been examined by analyzing genes which are expressing under various conditions, gene mutations in various individuals and the like. In addition, diagnoses of diseases and the like are now carried out using information on these genes.

Detection of mutations in nucleic acid sequences is very important in the field of medical genetics. Detection of genetic mutation is important, for example, in determining molecular biological grounds in hereditary diseases, providing carriers and prenatal diagnoses for genetic counseling, accelerating individualization regarding medicines, and identifying polymorphism for studies on genetics.

Detection and analysis of genetic mutation at DNA level have been carried out by the classification of karyotype and analysis of restriction fragment length polymorphism (RFLPs: Restriction Fragment Length Polymorphism) or variable numbers of tandem repeats (VNTRs: Variable Numbers of Tandem Repeats), or in recent years, by single nucleotide polymorphism (SNPs) analysis (Lai E. et al., Genomics, 15, 54 (1), pp. 31 - 38 (1998), Gu Z. et al., Hum. Mutat., 12 (4), pp. 221 - 225 (1998), Taillon-Miller P. et al., Genome Res., 8 (7), pp. 748 - 754 (1998), Weiss KM., Genome Res., 8 (7), pp. 691 - 697 (1998) and Zhao LP. et al., Am. J. Hum. Genet., 63 (1), pp. 225 - 240 (1998)). Markedly comprehensive techniques have so far been developed for the purpose of detecting and analyzing SNP (U.S. Patent No. 5,858,659, U.S. Patent No. 5,633,134, U.S. Patent No. 5,719,028, International Publication No. 98/30717, International Publication No. 97/10366, International Publication No. 98/44157, International Publication No. 98/20165, International Publication No. 95/12607 and International Publication No. 98/30883), but development of a more convenient and efficient method is in demand.

### Summary of the Invention

The invention aims at providing a method for efficiently detecting a mismatch between a single-stranded nucleic acid as the measuring object (target nucleic acid) and a single-stranded nucleic acid of known sequence (control nucleic acid).

With the aim of solving the aforementioned problems, the present inventors have conducted intensive studies and found as a result that the presence or absence of a mismatch between a control nucleic acid a target nucleic acid can be judged, not by measuring a mismatch binding protein bonded to a mismatch site through the immobilization or labeling of the mismatch binding protein as is conventionally known, but by effecting formation of a double-stranded nucleic acid through the hybridization of the control nucleic acid and target nucleic acid, allowing a mismatch binding protein to contact with said double-stranded nucleic acid and thereby to bind to a mismatch site, further allowing an intercalating agent which specifically recognizes the double-stranded nucleic acid to contact with said double-stranded nucleic acid, and then measuring amount of the intercalating agent intercalated into the double-stranded nucleic acid. The invention has been accomplished based on these findings.

That is, the invention consists of the following components.
<1> A method for detecting a mismatch between a target nucleic acid, which is a single-stranded nucleic acid, as a measuring object and a control nucleic acid, which is a single-stranded nucleic acid of known sequence, the method comprising:
   (a) effecting formation of a double-stranded nucleic acid through hybridization of the control nucleic acid and the target nucleic acid;
   (b) allowing a mismatch binding protein to contact with the double-stranded nucleic acid and thereby to bind to a mismatched site;
   (c) allowing an intercalating agent which specifically recognizes the double-stranded nucleic acid and is intercalated therein, to contact with the double-stranded nucleic acid;
   (d) detecting the intercalating agent intercalated into the double-stranded nucleic acid; and
   (e) judging the presence or absence of a mismatch between the control nucleic acid and the target nucleic acid, by comparing amounts of the intercalating agent intercalated into the double-stranded nucleic acid in the absence and presence of the mismatch binding protein.
<2> The method as described in <1> above,
   wherein the mismatch binding protein is MutS.
<3> The method as described in <1> or <2> above,
   wherein at least one of:
   1) a complementary probe comprising an oligonucleotide having a complementary nucleotide sequence moiety complementary to a predetermined nucleotide sequence moiety in a gene; and
   2) a partial complementary probe comprising an oligonucleotide having a partial complementary nucleotide sequence moiety wherein one or more bases in the complementary nucleotide sequence moiety are replaced by bases of other than the complementary nucleotide sequence moiety, is used as the control nucleic acid.
<4> The method as described in any of <1> to <3> above,
   wherein the intercalating agent which recognizes the double-stranded nucleic acid is a nucleic acid intercalator.
<5> The method as described in <4> above,
   wherein the nucleic acid intercalator is detected by a fluorescence method.
<6> The method as described in <4> or <5> above,
   wherein the nucleic acid intercalator has an electrochemically active region and is detected by a difference in current or voltage.
<7> The method as described in <6> above,
   wherein an electric potential is applied to an analytical element comprising a conductive substrate in the presence of the nucleic acid intercalator having an electrochemical activity, and a current value flowing between the intercalator and the analytical element is measured.
<8> The method as described in <7> above,
   wherein a current value flowing between the intercalator and the analytical element under a hybridization-bonded state of the complementary probe and the target nucleic acid is compared with a current value flowing between the intercalator and the analytical element under a hybridization-bonded state of the partial complementary probe and the target nucleic acid.
<9> The method as described in any of <1> to <8> above,
   wherein the target nucleic acid is a sample DNA fragment obtained from a sample gene.
<10> The method as described in any of <1> to <8> above,
wherein the target nucleic acid or the control nucleic acid is a product of a polymerase reaction.

### Detailed Description of the Invention

The invention relates to a method for detecting a mismatch between a control nucleic acid and a target nucleic acid making use of a mismatch binding protein. The method of the invention is a method which uses the ability of a mismatch binding protein to recognize a mismatch in a double-stranded nucleic acid and the property of an intercalating agent to recognize the double-stranded nucleic acid and thereby to be intercalated therein, and uses, as the index, a change in the amount of the intercalating agent intercalated into the double-stranded nucleic acid through binding of the mismatch binding protein to the double-stranded nucleic acid.

Accordingly, the method of the invention comprises (a) a step for effecting formation of a double-stranded nucleic acid through hybridization of the control nucleic acid and target nucleic acid, (b) a step for allowing a mismatch binding protein to contact with the double-stranded nucleic acid and thereby to bind to a mismatched site, (c) a step for allowing an intercalating agent which specifically recognizes the double-stranded nucleic acid and is intercalated therein, to contact with the double-stranded nucleic acid, (d) a step for detecting the intercalating agent intercalated into the double-stranded nucleic acid, and (e) a step for judging the presence or absence of a mismatch between the control nucleic acid and target nucleic acid, by comparing amounts of the intercalating agent intercalated into the double-stranded nucleic acid in the absence and presence of the mismatch binding protein.

A principle of the method of the invention is described in the following. A target nucleic acid having a possibility of possessing a mutation and a control nucleic acid (a nucleic acid which does not have mutation) are prepared, and they are hybridized with each other. As a result of this, when the target nucleic acid is possessed of a mutation, a heterogeneous double-stranded nucleic acid (a double-stranded nucleic acid having a mismatch) is formed by its hybridization with the control nucleic acid. On the other hand, when the target nucleic acid does not have a mutation, the heterogeneous double-stranded nucleic acid is not formed, but only a homogeneous double-stranded nucleic acid (a double-stranded nucleic acid having no mismatch) is formed.

When a mismatch binding protein is allowed to contact with the double-stranded nucleic acid formed by hybridization, the mismatch binding protein binds to a heterogeneous mismatch binding protein having a mismatch, but does not bind to the homogenous mismatch binding protein. In this case, when an intercalating agent that recognizes the double-strandednucleic acid and is thereby intercalated therein is allowed to contact with the double-stranded nucleic acid, the intercalating agent binds to the homogeneous double-stranded nucleic acid to which the mismatchbindingprotein is not bonded, but the intercalating agent is not intercalated into the heterogeneous double-stranded nucleic acid to which the mismatch binding protein is bonded.

Accordingly, whether or not the target nucleic acid has a mutation can be judged by detecting the intercalating agent intercalated into the double-stranded nucleic acid. That is, when a significant difference in the amount of the intercalating agent intercalated into the double-stranded nucleic acid is detected in the absence and presence of the mismatch binding protein, it is judged that a mutation is present in the target nucleic acid. On the other hand, when a significant difference in the amount of the intercalating agent intercalated into the double-stranded nucleic acid is not detected in the absence and presence of the mismatch binding protein, it is judged that a mutation is not present in the target nucleic acid.

According to the invention, the "mismatch" means that a set of base pair selected from adenine (A), guanine (G), cytosine (C) and thymine (T) (uracil (U) in the case of RNA) is not a normal base pair (A/T or G/C) . According to the invention, not only one mismatch but two or more continued mismatches, a mismatch caused by the insertion and/or deletion of one or two or more bases, and a combination thereof are included in the "mismatch".

According to the invention, the "mutation" means a base (a base pair in the case of double-stranded nucleic acid) in the target nucleic acid, which is different when compared with the control nucleic acid.

According to the invention, the "nucleic acid" includes DNA and RNA, such as a cDNA, a genomic DNA, an mRNA and a synthetic polynucleotide. It also includes a single-stranded nucleic acid and a double-stranded nucleic acid, and also a straight chain nucleic acid and a cyclic nucleic acid.

According to the invention, the "control nucleic acid" means a nucleic acid which does not have a mutation. Also, the "target nucleic acid" means a nucleic acid having a possibility of possessing a base which is different from that of the control nucleic acid (mutation). The target nucleic acid is a nucleic acid identical to the control nucleic acid when it does not have a mutation, and when it has a mutation, it is a nucleic acid wherein only said mutated region is different from the control nucleic acid. For example, when a mutation in a gene of a patient having a possibility of possessing a hereditary disease is detected, the gene of the patient having a possibility of possessing a mutation is the target nucleic acid, and a gene of a healthy person which corresponds to this gene is the control nucleic acid.

The target nucleic acid to be used in the method of the invention is not particularly limited, and any desired nucleic acid from which whether or not it has a mutation is to be detected can be used. In addition, a nucleic acid identical to the target nucleic acid is used as the control nucleic acid, with the proviso that it is a nucleic acid which corresponds to the target nucleic acid and the target nucleic acid does not have a mutation. This word "identical" means that both of them are identical to each other within the region to be hybridized, and though they may have different lengths, it is desirable to make them uniform when possible . The target nucleic acid and control nucleic acid may be single-stranded chains or double-stranded chains, but when both of them are single-stranded chains, they are chains complementary to each other with the proviso that the target nucleic acid does not have a mutation.

According to the method of the invention, the target nucleic acid and control nucleic acid are hybridized (however, when they are double-stranded chains, they are hybridized after dissociating them into single-stranded chains by denaturation). By this, a double-stranded nucleic acid is formed (the double-stranded nucleic acid becomes a mixture of heterogeneous double-stranded nucleic acid and homogeneous double-stranded nucleic acid when a mutation is present in the target nucleic acid, or becomes homogeneous double-stranded nucleic acid when a mutation is not present in the target nucleic acid).

As the method for denaturing double-strandednucleic acid, for example, a method in which pH of its solution is adjusted to an acidic or alkaline value and a method in which the solution is heated to a high temperature can be cited. As the method for changing pH value, for example, a method in which the solution is replaced with a 0.1 M NaOH or 0.1 M HCl solution can be cited. Also, regarding the method for increasing temperature, the solution may be set to a melting temperature (Tm) or more of the nucleic acid, but about 95°C is generally used.

Hybridization of two single-stranded nucleic acid chains can be easily carried out by returning pH of the solution to neutral level or gradually lowering the temperature to Tm or less. When it is presumed that the single-strandednucleic acids are remained in the process of forming the double-stranded nucleic acid, it is desirable for example to remove the single-stranded nucleic acids using a column or block the single-stranded nucleic acids in advance with an *Escherichia coli* SSB protein or the like.

The method of the invention can be suitably applied to the detection of a single mismatch base pair, two or more continued mismatches, mismatch of two or more bases of one base pair, and a mismatch generated by the deletion and/or insertion of one or two or more bases in at least one chain of the double-stranded nucleic acid.

The mismatch binding protein to be used in the method of the invention is a protein which recognizes a mismatch in a double-stranded nucleic acid and binds thereto, and its preferred examples include MutS, MSH2 and MSH6. Their origins have no particular limitation, with the proviso that they can recognize a mismatch in a double-stranded nucleic acid. Also, these proteins may be in the form of partial peptides, with the proviso that it can recognize a mismatch in a double-stranded nucleic acid. In addition, the mismatch binding protein may be a fusion protein with other protein, such as glutathione-S-transferase.

In addition, the mismatch binding protein may be a protein consisting of an amino acid sequence in which one or two or more amino acids in the natural type protein are substituted, deleted, added and/or inserted (a mutant), with the proviso that it can recognize mismatch in the double-stranded nucleic acid. Such a mutant is sometimes generated in the natural world, but it is possible to prepare it artificially by optionally making use of a conventionally known method.

It is possible to prepare the mismatch binding protein as a natural protein, or as a recombinant protein, by optionally combining conventionally known methods such as anion exchange column chromatography, cation exchange column chromatography, gel filtration column chromatography and ammonium sulfate fractionation. In addition, in the case of a recombinant protein having large expressed amount, it is also possible to prepare it more easily by a single chromatography which uses a cation exchange column and a gel filtration column.

Contact of a double-stranded nucleic acid with a mismatch binding protein in the method of the invention is carried out under such conditions that said protein can bind to a mismatch site in said double-stranded nucleic acid (e.g., appropriate pH, solvent, ionic environment and temperature). Detailed conditions of reaction temperature, salt concentration, kinds of ions, pH of a buffer and the like can be optionally adjusted.

The intercalating agent to be used in the invention is not particularly limited with the proviso that it can be intercalated by specifically recognizing a double-stranded nucleic acid, but is preferably a nucleic acid intercalator, more preferably a DNA intercalator. The nucleic acid intercalator may be a substance which by itself can form a detectable signal, but a signal forming substance may be linked to its side chain or to the intercalator via a specific binding pairsuch asbiotin-avidin,antigen-antibody or hapten-antibody. It is desirable that the detectable signal according to the invention is a signal detectable, for example, by fluorescence detection, luminescence detection, chemiluminescence detection, bioluminescence detection, electrochemical luminescence detection, radioactivity detection, electrochemical detection or colorimetric detection, though not particularly limited thereto.

As a preferable example of the nucleic acid intercalator, an intercalator itself may have a signal forming ability such as the case of a fluorescence dye, or it may be a complex of an intercalator with a signal forming substance. As the complex of an intercalator with a signal forming substance, for example, those of the following formulae (1) and (2) can be cited; formula (1) X-L1-I-L2-Y, formula (2) X-L1-I (in the formulae (1) and (2), I represents a substance which is intercalated into a double-stranded nucleic acid, L1 and L2 represent linker sequences, and X and Y represent detectable molecules).

In the formulae (1) and (2), the substance represented by I which is intercalated into a double-stranded nucleic acid is preferably a substance that has a phenyl group or the like flat plate shape intercalating group and can binds to the double-stranded nucleic acid through the intervention of said intercalating group between a base pair and a base pair of the double-stranded DNA.

In the formulae (1) and (2), the linker sequences represented by L1 and L2 are not particularly limited, and their examples include alkylene group, -O- group, -CO- group, -NH- group or a combination thereof.

In the formulae (1) and (2), illustrative examples of the detectable molecules represented by X and Y include a fluorescence dye group typified by fluorescein, Rhodamine, Cy5, Cy3, Texas Red, ruthenium complex and the like, a substance which forms a specific binding pair such as biotin-avidin, antigen-antibody or hapten-antibody, an electrochemically detectable substance typified by ferrocene derivatives, a luminescent substance suchas a lucigenin derivative or a luminol derivative and an enzyme which is used in so-called EIA (enzyme immunoassay).

When X and Y are substances which form a specific binding pair, a fluorescence dye group typified by fluorescein, Rhodamine, Cy5, Cy3, Texas Red, ruthenium complex and the like, an electrochemically detectable substance typified by ferrocene derivatives, a luminescent substance such as a lucigenin derivative or a luminol derivative and an enzyme which is used in so-called EIA (enzyme immunoassay) can be bonded via X and Y.

The nucleic acid intercalator to be used in the invention having an electrochemical activity is not particularly limited, with the proviso that it is intercalated by specifically recognizing a double-stranded nucleic acid and has an electrochemical activity, and its examples include a ferrocene compound, a catecholamine compound, a metal bipyridine complex, a metal phenanthrene complex, a viologen compound and the like. Particularly preferred is a ferrocene modified tuck type intercalator.

As the intercalating agent to be used in the invention, for example, ethidium, ethidium bromide, acridine, aminoacridine, acridine orange, bisbenzimide, diaminophenylindole, proflavine, ellipticine, actinomycin D, thiazole, chromomycin, daunomycin, mitomycin C and derivatives thereof can also be used. In addition, those which are described in JP-A-62-282599 can be exemplified as other available intercalating agents.

There is no limitation to the method for detecting an intercalating agent intercalated into a double-stranded nucleic acid formed by the hybridization of a control nucleic acid with a target nucleic acid in the presence of a mismatch binding protein. For example, the following detection systems can be considered.

According to the method of the invention, (1) a double-stranded nucleic acid formed by the hybridization of a control nucleic acid with a target nucleic acid is used by immobilizing it on a support, a mismatch binding protein is allowed to contact with the double-stranded nucleic acid, the mismatchbindingproteinnot bonded to the nucleic acid is removed, and then an intercalating agent is allowed to contact with the double-stranded nucleic acid to detect the intercalating agent intercalated into the double-stranded nucleic acid. (2) A mismatch binding protein is allowed to contact with a double-stranded nucleic acid formed by immobilizing a target nucleic acid immobilized on a support and hybridizing it with a control nucleic acid, the mismatch binding protein not bonded to the nucleic acid is removed, and then an intercalating agent is allowed to contact with the double-stranded nucleic acid to detect the intercalating agent intercalated into the double-stranded nucleic acid. (3) A mismatch binding protein is allowed to contact with a double-stranded nucleic acid formed by immobilizing a control nucleic acid immobilized on a support and hybridizing it with a target nucleic acid, the mismatch binding protein not bonded to the nucleic acid is removed, and then an intercalating agent is allowed to contact with the double-stranded nucleic acid to detect the intercalating agent intercalated into the double-stranded nucleic acid.

As the support, it may be any support which can effect solid-liquidseparation, such as a membrane filter, amicrotiter plate, a chromatography carrier, magnetic beads, a conductive substrate, a glass plate, a plastic plate or the like.

For example, as is described in JP-A-2003-75402, at least any one of a control nucleic acid, a target nucleic acid, a complementary probe and a partial complementary probe samples, or a sample DNA fragment, is hybridized with said control nucleic acid, target nucleic acid, sample DNA fragment, complementary probe or partial complementary probe, on an analytical element prepared by immobilizing at least any one of the control nucleic acid, target nucleic acid, sample DNA fragment, complementary probe and partial complementary probe on a conductive substrate, the thus hybridized double-stranded nucleic acid is allowed to contact with a mismatch binding protein and thereby to bind to a mismatch site, subsequently, a nucleic acid intercalator having an electrochemical activity is allowed to contact with the double-stranded nucleic acid to effect intercalation of said nucleic acid intercalator into the double-stranded nucleic acid, and then the current value flowing between said intercalator and analytical element is measured.

In addition, it is desirable to compare the current value flowing between said intercalator and analytical element under a hybridization-bonded state of the complementary probe and a sample DNA fragment prepared from a sample gene, with the current value flowing between said intercalator and analytical element under a hybridization-bonded state of the partial complementary probe and a sample DNA fragment prepared from a sample gene.

The method of the invention can be used for examining whether or not a gene derived from a patient and the gene of a healthy person have the same nucleotide sequence, in order to examine whether or not a specific gene has a mutation in a patient having a possibility of getting a hereditary disease. The method of the invention can detect a mutation regardless of its position in the target gene and is also superior because it is not necessary that the mutation site and kind of the mutation in the gene to be inspected are conventionally known.

### [Examples]

### [Inventive Example 1]

### (1) Preparation of a DNA fragment detecting tool

Each of the following two oligonucleotides (1 x 10⁻⁶ M) having aminohexyl group on the 5' terminus was dispersed in 0.1 M carbonate buffer (pH 9.3), and 1 µl of the aqueous dispersion was spotted on spot A or B on a solid phase carrier prepared by introducing vinylsulfonyl group onto the surface of a slide glass via a silane coupling agent (mfd. by Shin-Etsu Silicon) and then allowed to stand at a humidity of 75% for 18 hours, thereby preparing a DNA fragment detecting tool.
Spot A: 5'-GATCAGACACTTCAAGGTCTAGG-3' (SEQ ID NO:1)
Spot B: 5'-GATCAGACAATTCAAGGTCTAGG-3' (SEQ ID NO:2)

The aforementioned two oligonucleotides were designed such that they are identical to each other except for the underlined one base, and an oligonucleotide of the standard sequence was fixed to the spot A, and that of a comparative sequence 1 to the spot B, respectively. The sign I represents deoxyinosine.

### (2) Preparation of sample DNA fragment

As the sample DNA fragment, a DNA fragment of the following sequence (a sequence completely complementary to the oligonucleotide of the standard sequence, to be regarded as a normal sequence) was prepared.
Sample: 5'-CTAGTCTGTGAAGTTCCAGATCC-3' (SEQ ID NO:3)

### (3) Hybridization

A dispersion prepared by dispersing the sample DNA fragment of (2) (1 x 10⁻⁶ M) in 20 µl of a hybridization solution [a mixed solution of 4 x SSC (mfd. by Invitrogen) and a 10% by weight SDS aqueous solution] was spotted on the DNA fragment detecting tool. Thereafter, 1 µl of Taq-MutS (mfd. by Nippon Gene) (1 µg/µl) was added to the tool, its surface was protected with a cover glass for microscope use, and then this was incubated at 60°C for 2 hours in a Tupperware. Subsequently, the cover glass was removed, and the slide glass was soaked in a SyberGreen solution (mfd. by Molecular Probe, 1,000 times dilution TE solution) for 20 minutes and then washed with TE (mfd. by Invitrogen, pH 8.0).

### (4) Measurement of fluorescence intensity

Fluorescence intensity (relative value) of the thus obtained spotted parts of the DNA fragment detecting tool was measured using a fluorescence scanning device (FLA 8000, mfd. by Fuji Photo Film).

### [Inventive Example 2]

Hybridization and measurement of fluorescence intensity were carried out in the same manner as in Inventive Example 1, except that a DNA fragment of the following sequence (a sequence completely complementary to the oligonucleotide of the comparative sequence 1, to be regarded as an abnormal sequence) was prepared as the sample DNA fragment in Inventive Example 1(2).
Sample: 5'-CTAGTCTGTTAAGTTCCAGATCC-3' (SEQ ID NO:4)

### [Inventive Example 3]

Hybridization and measurement of fluorescence intensity were carried out in the same manner as in Inventive Example 1, except that a DNA fragment of the following sequence (a sequence in which only 1 base in the normal sequence is mutated, to be regarded as an abnormal sequence) was prepared as the sample DNA fragment in Inventive Example 1(2).
Sample: 5'-CTAGTCTGTCAAGTTCCAGATCC-3' (SEQ ID NO:5)

### [Results]

The results obtained in Inventive Examples 1 to 3 are shown in Table 1. Since the fluorescence intensity was spot A > spot B in Inventive Example 1 as shown in Table 1, it was able to judge that the sample DNA fragment is a DNA fragment of normal sequence. Since the fluorescence intensity was spot A < spot B in Inventive Example 2, it was able to judge that the sample DNA fragment is a DNA fragment of abnormal sequence (the substituted base is T). Also, since the fluorescence intensity was spot A ≒ spot B also in Inventive Example 3, it was able to judge that the sample DNA fragment is a DNA fragment of abnormal sequence (the substituted base is unspecified).

**Table 1**

| Sample DNA fragments | | Fluorescence intensity |
|---|---|---|
| Inventive Example 1 | Spot A | 3200 |
| | Spot B | 1760 |
| Inventive Example 2 | Spot A | 1780 |
| | Spot B | 3500 |
| Inventive Example 3 | Spot A | 1800 |
| | Spot B | 1790 |

### [Comparative Examples 1 to 3]

As comparative examples, the same operations of Inventive Examples 1 to 3 were carried out, except that 1 µl of Taq-MutS (1 µg/µl) was not added in the operation of (3) Hybridization. The thus obtained results are shown in Table 2. In the comparative examples 1, 2 and 3 shown in Table 2, the fluorescence intensity of all spots was spot A ≒ spot B, so that it was unable to determine the presence or absence of a mutation in the target nucleic acid. Thus, it was found from the results of inventive examples and comparative examples that whether or not a mutation is formed at the aimed site of a target nucleic acid, namely whether or not the target nucleic acid is a normal DNA fragment identical to the control nucleic acid or has a single nucleotide polymorphism, can be judged by the invention.

**Table 2**

| Sample DNA fragments | | Fluorescence intensity |
|---|---|---|
| Comparative Example 1 | Spot A | 3500 |
| | Spot B | 3300 |
| Comparative Example 2 | Spot A | 2900 |
| | Spot B | 3100 |
| Comparative Example 3 | Spot A | 2800 |
| | Spot B | 3100 |

### [Production Example 1]

### Production of N,N'-bis(7-ferrocene carboxylate acido-4-methyl-4-azaheptyl)naphthalenediimide (1) Production of N-1-benzyloxycarbonyl-1,7-diamino-4-methyl-azaheptane

Di(3-aminopropyl)-N-methylamine (73.0 g, 500 mmol) was dissolved in dichloromethane (400 ml), and a dichloromethane (100 ml) solution of 3-benzyloxycarbonyl-1,3-thiazoline-2-thion (Synthesis, 1990, 27) (12.8 g, 50 mmol) was added dropwise thereto and stirred at room temperature for 3 hours. Next, the thus formed precipitate was separated by filtration, and the filtrate was mixed with ethyl acetate and water and extracted twice with ethyl acetate. The ethyl acetate layer was washed with water and saturated aqueous solution and then extracted twice with 1 N hydrochloric acid aqueous solution, and the thus obtained water layer was washed with ethyl acetate. While cooling, the water layer was adjusted to pH 9 to 10 by adding 6 N sodium hydroxide aqueous solution thereto and extracted with ethyl acetate. The ethyl acetate layer was washed with saturated brine and dried with anhydrous sodium sulfate, and then the solvent was evaporated to obtain the title compound as a yellow oily substance (9.4 g, yield 66%). ¹H-NMR (300 MHz, CDCl₃) δ; 1.58 - 1.72 (4 H, m), 2.20 (3 H, s), 2.35 - 2.45 (4 H, m), 2.64 (2 H, t), 3.23 - 3.32 (2 H, m), 5.15 (2 H, s), 7.22 - 7.45 (5 H, m) MS: FAB 280 (M⁺ + 1) (matrix: m-nitrobenzene)

### (2) Production of N-1-benzyloxycarbonyl-1-amino-7-ferrocene carboxylate acido-4-methyl-4-azaheptane

The N-1-benzyloxycarbonyl-1,7-diamino-4-methyl-azaheptane obtained in the aforementioned (1) (3.0 g, 11 mmol) was dissolved in dichloromethane (30 ml), and ferrocene carboxylate (2.5 g, 11 mmol), pyridine (2 ml) and N,N'-dimethylaminopropylcarbodiimide hydrochloride (2.3g, 12 mmol) were added thereto and stirred at room temperature for 3 hours. The reaction solution was mixed with an ammonium chloride aqueous solution and extracted twice with ethyl acetate, the ethyl acetate layer was washed with saturated brine, and then the solvent was evaporated. The thus obtained brown oily substance was subjected to an alumina column chromatography (eluting solvent; chloroform:methanol = 20:1), and the thus obtained crystals were washed with a mixed solvent of hexane-ethyl acetate to obtain the title compound as orange crystals (3.3 g, yield 62%).
¹H-NMR (300 MHz, CDCl₃) δ; 1.62 - 1.90 (4 H, m), 2.27 (3 H, s), 2.40 - 2.62 (4 H, m), 3.25 - 3.39 (2 H, m), 3.39 - 3.58 (2 H, m), 4.22 (5 H, s), 4.31 (2 H, s), 4.69 (2 H, s), 5.14 (2 H, s), 5.60 (1 H, bs), 6.82 (1 H, bs), 7.27 - 7.48 (5 H, m)

### (3) Production of 1-amino-7-ferrocene carboxylate acido-4-methyl-4-azaheptane

The N-1-benzyloxycarbonyl-1-amino-7-ferrocene carboxylate acido-4-methyl-4-azaheptane obtained in the aforementioned. (2) (1.5 g, 3.0 mmol) was dissolved in acetonitrile (30 ml), and while stirring at room temperature, trimethylsilane iodide (1.25 ml, 8.8 mmol) was added dropwise thereto. Five minutes thereafter, the reaction solution was mixed with 1 N hydrochloric acid aqueous solution and ethyl acetate and extracted three times with 1 N hydrochloric acid aqueous solution, and the water layer was washed with ethyl acetate. The water layer was ice-cooled, adjusted to pH 10 by adding 2 N potassium hydroxide aqueous solution and extracted twice with chloroform. The organic layer was washed with saturated brine, and then the solvent was evaporated to obtain the title compound as brown crystals (1.0 g, yield 93%).
¹H-NMR (300 MHz, CDCl₃) δ; 1.57 - 1.87 (4 H, m), 2.33 (3 H, s), 2.41 - 2.60 (4 H, m), 2.86 (2 H, t), 3.40 - 3.53 (2 H, m), 4.24 (5 H, s), 4.37 (2 H, s), 4.70 (2 H, s)

### (4) Production of N,N'-bis(7-ferrocene carboxylate acido-4-methyl-4-azaheptyl)naphthalenediimide

The 1-amino-7-ferrocene carboxylate acido-4-methyl-4-azaheptane obtained in the aforementioned (3) (0.9 g, 2.5 mmol) was dissolved in tetrahydrofuran (50 ml), and while stirring at room temperature, naphthalene-1,4,5,8-tetracarboxylic acid dianhydride (0.3 g, 1.1 mmol) was added thereto and refluxed for 7 hours. The reaction solution was filtered and then washed with chloroform, the solvent was evaporated from the combined organic layer, the thus obtained residue was subjected to an alumina column chromatography (eluting solvent; chloroform:methanol = 5:1), and the thus obtained crystals were washed with ethyl acetate, thereby obtaining the title compound as brown crystals (0.66 g, yield 62%) .
¹H-NMR (300 MHz, CDCl₃) δ; 1.70 - 1.85 (8 H, m), 1.93 - 2.09 (4 H, m), 2.35 (6 H, s), 2.51 - 2.66 (8 H, m), 3.45 - 3.56 (4 H, m), 4.19 (10 H, s), 4.32 (4 H, s), 4.70 (4 H, s), 7.19 (2 H, bs), 8.79 (4 H, s)
MS: FAB 947 (M + H) (matrix: m-nitrobenzene)

### [Inventive Example 4]

### (1) Preparation of electrochemical analysis element

An aqueous solution (2 µl) of 100 pmol/l µl of thymine pentadecamer (dT₁₅) having mercaptohexyl group on the 5' end was added dropwise to a metal electrode plate having an area of 2.25 mm², and this was allowed to stand at room temperature for 1 hour to prepare an electrochemical analysis element. In this connection, preparation and immobilization of dT₁₅ were carried out in accordance with the method described in JP-A-9-288080.

### (2) Preparation of sample DNA fragment

A pentadecamer of adenine (dA₁₅) was prepared as the sample DNA fragment in accordance with the method described in the aforementioned document.

### (3) Detection of hybrid DNA

A 2 µl portion of 10 mM Tris buffer (pH 7.5) containing the dA₁₅ obtained in the aforementioned (2) (70 pmol) was added dropwise to the electrochemical analysis element prepared in the aforementioned (1), and this was incubated at 25°C for 20 minutes. After the incubation, unreacted dA₁₅ was removed by washing the analytical element surface with 0.1 M sodium dihydrogenphosphate-disodium hydrogenphosphate aqueous solution (pH 7.0) . Next, the analytical element after washing was soaked in a 0.1 M potassium chloride-0.1 M acetate buffer (pH 5.6) mixed solution containing the compound obtained in Production Example 1 [N,N'-bis(7-ferrocene carboxylate acido-4-methyl-4-azaheptyl)naphthalenediimide; a nucleic acid intercalator having an electrochemical activity] (50 µM), and measured by differential pulse voltammetry (DPV) under conditions of 50 mV in pulse amplitude, 50 ms in pulse width, a range of from 100 to 700 mV in applied voltage and 100 mV/second in scanning rate. Current capacity at a response potential of 460 mV was calculated. In addition, when a current capacity obtained by carrying out the same operation of the above except that the sample DNA fragment dA₁₅ was not added was regarded as the basal value, and the changed amount from the basal value of the current capacity obtained by the aforementioned measurement was calculated, it was 42%.

### [Inventive Example 5] (Detection of hybrid DNA having a mismatch structure)

### (1) Preparation of electrochemical analysis element

An electrochemical analysis element was prepared in the same manner as in Inventive Example 4(1), except that dT₁₄G₁ was used.

### (2) Detection of a hybrid DNA having a mismatch structure

The same operation of Inventive Example 4 was carried out, except that the electrochemical analysis element prepared in Inventive Example 4(1) was used as the electrochemical analysis element, 1 µg of Taq-MutS was used as the mismatch binding protein and the analytical element of the aforementioned element was used, respectively. When DPV was measured at an applied voltage of within the range of from 400 to 700 mV, and rate of change of the current capacity at 460 mV was calculated, it was 11%.

### [Comparative Example 4]

The same operation and measurement of Inventive Example 5 were carried out except that 1 µg of Taq-MutS was not used. When rate of change of the current capacity was calculated, it was 36%.

It can be seen from Inventive Example 4, Inventive Example 5 and Comparative Example 4 that the hybrid DNA obtained by allowing the sample DNA fragment dA₁₅ to contact with the electrochemical analysis element prepared by immobilizing dT₁₄G₁ is a mismatch structure hybrid DNA, and that a difference in the response current between a full-match structure hybrid DNA and a mismatch structure hybrid DNA can be obtained by the use of a mismatch binding protein Taq-MutS.

According to the method of the invention which uses Taq-MutS of a mismatch binding protein, the presence or absence of a mismatch between a control nucleic acid and a target nucleic acid can be detected conveniently with high sensitivity.

In addition, regarding the detection of nucleic acid fragments by an electrochemical technique, detection of SNPs can be easily carried out without employing fluorescence labeling and the like complex operations.

The invention described herein can be applied to genetic diagnosis, infection diagnosis, genome-based drug discovery and the like uses.

The entire disclosure of each and every foreign patent application from which the benefit of foreign priority has been claimed in the present application is incorporated herein by reference, as if fully set forth.

## Claims

1. A method for detecting a mismatch between a target nucleic acid, which is a single-stranded nucleic acid, as a measuring object and a control nucleic acid, which is a single-stranded nucleic acid of known sequence, the method comprising:
(a) effecting formation of a double-stranded nucleic acid through hybridization of the control nucleic acid and the target nucleic acid;
(b) allowing a mismatch binding protein to contact with the double-stranded nucleic acid and thereby to bind to a mismatched site;
(c) allowing an intercalating agent which specifically recognizes the double-stranded nucleic acid and is intercalated therein, to contact with the double-stranded nucleic acid;
(d) detecting the intercalating agent intercalated into the double-stranded nucleic acid; and
(e) judging the presence or absence of a mismatch between the control nucleic acid and the target nucleic acid, by comparing amounts of the intercalating agent intercalated into the double-stranded nucleic acid in the absence and presence of the mismatch binding protein.

2. The method according to claim 1,
wherein the mismatch binding protein is MutS.

3. The method according to claim 1,
wherein at least one of:
1) a complementary probe comprising an oligonucleotide having a complementary nucleotidesequence moiety complementary to a predetermined nucleotide sequence moiety in a gene; and
2) a partial complementary probe comprising an oligonucleotide having a partial complementary nucleotide sequence moiety wherein one or more bases in the complementary nucleotide sequence moiety are replaced by bases of other than the complementary nucleotide sequence moiety, is used as the control nucleic acid.

4. The method according to claim 1,
wherein the intercalating agent which recognizes the double-stranded nucleic acid is a nucleic acid intercalator.

5. The method according to claim 4,
wherein the nucleic acid intercalator is detected by a fluorescence method.

6. The method according to claim 4,
wherein the nucleic acid intercalator has an electrochemically active region and is detected by a difference in current or voltage.

7. The method according to claim 6,
wherein an electric potential is applied to an analytical element comprising a conductive substrate in the presence of the nucleic acid intercalator having an electrochemical activity, and a current value flowing between the intercalator and the analytical element is measured.

8. The method according to claim 7,
wherein a current value flowing between the intercalator and the analytical element under a hybridization-bonded state of the complementaryprobe and the target nucleic acid is compared with a current value flowing between the intercalator and the analytical element under a hybridization-bonded state of the partial complementary probe and the target nucleic acid.

9. The method according to claim 1,
wherein the target nucleic acid is a sample DNA fragment obtained from a sample gene.

10. The method according to claim 1,
wherein the target nucleic acid or the control nucleic acid is a product of a polymerase reaction.


SEQUENCE LISTING

<110>  Fuji Photo Film Co. Ltd.,

<120>  Detection of SNPs by fluorescent intercalater

<130>  206-02176

<160>  5

<170>  PatentIn version 3.1

<210>  1
<211>  23
<212>  DNA
<213>  Artificial

<220>
<223>  Artificially synthesized oligonucleotide sequence

<400>  1
gatcagacac ttcaaggtct agg                                                    23

<210>  2
<211>  23
<212>  DNA
<213>  Artificial

<220>
<223>  Artificially synthesized oligonucleotide sequence

<400>  2
gatcagacaa ttcaaggtct agg                                                    23

<210>  3
<211>  23
<212>  DNA
<213>  Artificial

<220>
<223>  Artificially synthesized oligonucleotide sequence

<400>  3
ctagtctgtg aagttccaga tcc                                                    23

<210>  4
<211>  23
<212>  DNA
<213>  Artificial

<220>
<223>  Artificially synthesized oligonucleotide sequence

<400>  4
ctagtctgtt aagttccaga tcc                                                    23


<210>  5
<211>  23
<212>  DNA
<213>  Artificial

<220>
<223>  Artificially synthesized oligonucleotide sequence

<400>  5
ctagtctgtc aagttccaga tcc                                                    23